## Europäisches Patentamt
## European Patent Office
## Office européen des brevets

(19)

(11) Veröffentlichungsnummer: **0 135 102**
**B1**

(12)
# EUROPÄISCHE PATENTSCHRIFT

(45) Veröffentlichungstag der Patentschrift:
**01.06.88**

(51) Int. Cl.⁴: **C 07 D 277/78**

(21) Anmeldenummer: **84109294.3**

(22) Anmeldetag: **06.08.84**

(54) Verfahren zur Herstellung von 2,2'-Dibenzothiazolyldisulfid.

(30) Priorität: **19.08.83 DE 3329976**

(43) Veröffentlichungstag der Anmeldung:
**27.03.85 Patentblatt 85/13**

(45) Bekanntmachung des Hinweises auf die Patenterteilung:
**01.06.88 Patentblatt 88/22**

(84) Benannte Vertragsstaaten:
**BE DE FR GB IT NL**

(56) Entgegenhaltungen:
**DE - A - 2 309 584**
**DE - A - 2 800 462**
**US - A - 2 468 952**

(73) Patentinhaber: **BAYER AG, Konzernverwaltung RP Patentabteilung, D-5090 Leverkusen 1 Bayerwerk (DE)**

(72) Erfinder: **Wüst, Alfredo, Dr., Siebengebirgsweg 14, D-5064 Rösrath (DE)**

## Beschreibung

Das ökonomisch wichtigste Verfahren zur Herstellung von 2,2'-Dibenzothiazolyldisulfid ist die Oxidation von wässrigen Alkalimetallsalzlösungen des 2-Mercaptobenzothiazols mit Chlor oder Chlor/Luft-Gemischen. Zur Erzielung grosser Ausbeuten und befriedigender Reinheiten müssen bei dieser Reaktion bestimmte Rahmenbedingungen eingehalten werden, denn es besteht die Gefahr, durch Überoxidation sauerreagierende Nebenprodukte zu erzeugen, insbesondere bei hohen pH-Werten. Wird andererseits der pH-Wert zu niedrig, beispielsweise unter 7, fällt Mercapobenzthiazol aus, das auf Grund seiner geringen Wasserlöslichkeit kaum oxidiert wird und ebenfalls das gewünschte Endprodukt verunreinigt.

Um diese Nachteile zu vermindern, schlagen die Deutschen Offenlegungsschriften 2 309 584 und 2 800 462, beide von American Cyanamid Co. vor, unter die Oberfläche einer wässrigen Mischung, die durch einen Rührer intensiv gerührt wird, kontinuierlich getrennte Ströme aus einer wässrigen Lösung eines Alkalimetallsalzes von Mercaptobenzothiazol, aus einer wässrigen Lösung eines Alkalimetallhydroxids und aus gasförmigem Chlor einzuleiten, wobei die Temperatur 20 bis 75°C, der pH-Wert 6 bis 10 und das Redoxpotential der wässrigen Mischung −150 bis +250 mV betragen soll, und die Reaktion so geführt wird, dass die Konzentration der Reaktanden während der gesamten Umsetzungszeit praktisch Null ist. Bei diesem Verfahren wird angestrebt, Ausbeuten von über 97% zu erzielen und nicht mehr als 20% überschüssiges Chlor zu verbrauchen. Aus den Ausführungsbeispielen geht hervor, dass 10 bis 20 Mol-% überschüssiges Chlor und 10 bis 20 Mol-% Alkali, jeweils bezogen auf das Alkalisalz des 2-Mercaptobenzothiazol, verbraucht werden und dass trotz der komplizierten Reaktionsführung 1,7 bis 2,9 Mol-% Überoxidationsprodukte erzeugt werden und das gewünschte Produkt bis zu 1,1 Gew.% freies Mercaptobenzthiazol enthält.

Aufgabe der Erfindung war es, ein Oxidationsverfahren von wässrigen Alkalilösungen des 2-Mercaptobenzothiazols mittels Chlor zu finden, bei dem ein weitgehend von sauren Nebenprodukten und 2-Mercaptobenzothiazol freies 2,2'-Dibenzothiazolyldisulfid erhalten wird.

Die Aufgabe wird dadurch gelöst, dass man die Oxidation so führt, dass am Ende stets noch wenigstens 2,5 Gew.% des Alkalimetallsalzes des 2-Mercaptobenzothiazols im Reaktionsgemisch vorhanden sind.

Gegenstand der Erfindung ist daher ein Verfahren zur Herstellung von 2,2'-Dibenzothiazolyldisulfid durch Oxidation von wässrigen, 8 bis 50 gew.%igen Alkalimetallsalzlösungen des 2-Mercaptobenzothiazols mit Chlor oder Chlor/Luft-Gemischen bei Temperaturen von 15 bis 60°C und pH-Werten von 9 bis 13, das dadurch gekennzeichnet ist, dass die Konzentration des Alkalimetallsalzes des 2-Mercaptobenzothiazols zu keinem Zeitpunkt der Reaktion weniger als 2,5 Gew.% beträgt.

Die Reaktion kann ansatzweise oder kontinuierlich durchgeführt werden. Bei kontinuierlicher Reaktion beträgt die stationäre Konzentration (=Mindestkonzentration) an Mercaptobenzothiazol-Alkalimetallsalz vorzugsweise 5 bis 25 Gew.%.

Bei ansatzweiser Fahrweise beträgt die Konzentration des Alkalimetallsalzes des 2-Mercaptobenzothiazols am Ende der Reaktion vorzugsweise 5 bis 9 Gew.%.

Besonders gute Ergebnisse werden erzielt, wenn der pH-Wert der Reaktionsmischung 10 bis 12,5 beträgt. Dies ist überraschend, da nach den genannten Deutschen Offenlegungsschriften vor pH-Werten über 10 eindringlich gewarnt wird.

Unter den vorstehend genannten Bedingungen ist es möglich, den Gehalt an sauren Nebenprodukten unter die Nachweisgrenze zu drücken und ein lagerstabiles und thermisch stabiles 2,2'-Dibenzothiazolyldisulfid in hohen Ausbeuten mit hervorragender Reinheit zu erhalten, das nur durch geringste Mengen 2-Mercaptobenzothiazol verunreinigt ist. In günstigen Fällen gelingt es ferner, den Chlorverbrauch auf einen Überschuss von maximal 5 Mol.-% und den Verbrauch an Alkalimetallhydroxid ebenfalls auf maximal 5 Mol-% zu begrenzen.

Nicht umgesetzte Alkalimetallsalze des 2-Mercaptobenzothiazols verbleiben beim Abfiltrieren des gewünschten Produktes in der wässrigen Lösung und können durch Ansäuern als 2-Mercaptobenzothiazols ausgefällt, isoliert und weiterbzw. wiederverwendet werden.

Vorzugsweise setzt man das Natriumsalz des 2-Mercaptobenzothiazols ein und hält den pH-Wert mittels Natronlauge im gewünschten Bereich.

In den nachfolgenden Tabellen haben die einzelnen Spalten folgende Bedeutungen:

A: pH-Wert
B: Anfangskonzentration an NaMBT in Gew.%
C: Endkonzentration an NaMBT in Gew.%
D: Ausbeute in %, bezogen auf umgesetzter NaMBT
E: Chlorverbrauch in Mol-% bezogen auf NaMBT
F: NaOH-Verbrauch in Mol-% bezogen auf NaMBT
G: Gehalt an 2,2'-Dibenzothiazolyldisulfid in Gew.%
H: Gehalt an sauren Verbindungen in Gew.% bezogen auf isoliertes Produkt
I: Gehalt an MBT in Gew.% bezogen auf isoliertes Produkt

### Beispiel 1

In einem thermostatisierten Doppelmantel-Planschliffgefäss von 3 l Inhalt mit KPG-Rührer, Gaseinleitungsfritte, pH-Messkette, Thermometer, Tropftrichter und Rückflusskühler werden 756 g einer 50 gew.%igen, durch dreimaliges fraktioniertes Umfällen mit Salzsäure und Wiederauflösen der Mittelfraktion in Natronlauge gereinigten Lösung des 2-Mercaptobenzothiazol-Natriumsalzes (NaMBT) und 1248 g entsalztes Wasser vorgelegt. Die Lösung ist 18,9 gew.%ig an NaMBT.

Bei 35°C wird ein konstanter Gasstrom eines Chlor-Luft-Gemisches aus 10 l/h Chlor und 200 l/h Luft eingeleitet. Gleichzeitig werden 344 g/h der 50 gew.%igen NaMBT-Lösung zugegeben, wodurch die NaMBT-Konzentration konstant auf 18,9 Gew.% gehalten wird. Der pH-Wert wird durch Zugabe von 10 gew.%iger Natronlauge bei 10 konstant gehalten. Nach Einleiten von 16 g Chlor wird der Versuch beendet. Das ausgefallene 2,2'-Dibenzothiazolyldisulfid wird abgesaugt, neutral gewaschen und im Vakuum getrocknet.

Die Ausbeute beträgt 74 g, entsprechend 99,8% d.Th. bezogen auf eingesetztes NaMBT. Der Chlorverbrauch beträgt 101,2 Mol-%., der Verbrauch an Natronlauge 1 Mol-%, bezogen auf eingesetztes NaMBT. Der Wirkstoffgehalt beträgt 98,5%, der Gehalt an sauren Bestandteilen (ausgedrückt als Benzothiozolsulfonsäure) 0%, der Gehalt an freiem Mercaptobenzothiazol (MBT) 1,4%, Schmelzpunkt 170,2–172,8°C.

Der gleiche Versuch, bei pH 12 durchgeführt, ergibt folgende Ergebnisse:

Ausbeute: 99%; Chlorverbrauch: 104,8%; Natronlaugeverbrauch: 12%; Wirkstoffgehalt: 99,8%, Säuregehalt: 0%, Gehalt an freiem MBT: 0,2%; Schmelzpunkt: 174,9–177,8°C.

**Beispiel 2 bis 4**

Gemäss Beispiel 1 wird durch Vorlegen und Zugeben entsprechender Mengen NaMBT-Lösung jeweils eine konstante NaMBT-Konzentration im Reaktionsgefäss eingestellt und gehalten. Der pH-Wert wird durch Zugabe von Natronlauge konstant gehalten. Chlor und Luft werden als Gemisch mit konstanter Geschwindigkeit eingeleitet.

Je nach eingestellter Konzentration werden folgende Ausbeuten und Qualitäten erhalten:

| Beispiel | A | B/C | D | E | F | G | H | I |
|---|---|---|---|---|---|---|---|---|
| 2 (a) | 10 | 8 | 98,9 | 104,0 | 1 | 99,1 | 0 | 0,8 |
| (b) | 12 | 8 | 98,5 | 107,0 | 14 | 99,7 | 0 | 0,1 |
| 3 (a) | 10 | 1,9 | 98,3 | 106,4 | 3 | 99,7 | 0,1 | 0,2 |
| (b) | 12 | 1,9 | 97,8 | 108,8 | 19 | 99,6 | 0,3 | 0 |
| 4 (a) | 10 | 0,2 | 95,7 | 112,8 | 11 | 98,5 | 0,7 | 0,2 |
| (b) | 12 | 0,2 | 94,5 | 133,7 | 59 | 97,2 | 0,9 | 0,2 |

**Beispiel 5**

In einem 800 l Emaillekessel mit Rührwerk, Chloreinleitungsvorrichtung, Zulaufgefäss für Natronlauge und Umpumpvorrichtung mit pH-Messstelle werden 195 kg 50 gew.%iger NaMBT-Lösung und 455 l entsalztes Wasser, entsprechend einer 15 gew.%igen Lösung, vorgelegt und auf 35°C erwärmt. Unter Rühren leitet man bei pH 10 0,94 Nm³/h Chlor und 15,2 Nm³/h Luft als Gemisch ein. Innerhalb 5,2 Stunden werden 15,4 kg Chlor eingeleitet. Das Dibenzothiazolyldisulfid wird abfiltriert, chloridfrei gewaschen und getrocknet. Die Mutterlauge wird mit Salzsäure auf pH 3 angesäuert, das ausfallende 2-Mercaptobenzothiazol wird abgesaugt, chloridfrei gewaschen und getrocknet. Die Endkonzentration an NaMBT beträgt 2,5 Gew.%.

Es werden 69,9 kg 2,2'-Dibenzothiazolyldisulfid und 14,0 kg 2-Mercaptobenzothiazol, entsprechend einem Umsatz von 83,3%, erhalten. Ausbeute: 98%; Chlorverbrauch: 103 Mol-%, Natronlaugeverbrauch: 2 Mol-%. Wirkstoffgehalt: 98,8%; Säuregehalt: 0%; Gehalt an freiem MBT: 1,2%; Schmelzpunkt: 173,4–176,7°C.

**Beispiel 6**

In der Apparatur nach Beispiel 5 werden 50 kg 50 gew.%iger NaMBT-Lösung und 450 l entsalztes Wasser (5 Gew.% NaMBT) vorgelegt und auf 35°C erwärmt. Unter Rühren werden innerhalb 2 Stunden 5,2 kg Chlor (als Chlor/Luft-Gemisch) eingeleitet. Die Endkonzentration an NaMBT beträgt 0,04 Gew.%.

Nach Aufarbeitung wie in Beispiel 5 werden 21,2 kg 2,2'-Dibenzothiazolyldisulfid und 0,15 kg 2-Mercaptobenzothiazol entsprechend einem Umsatz von 99,2% erhalten. Ausbeute: 97,7%; Chlorverbrauch: 114 Mol-%; Natronlaugeverbrauch: 9 Mol-%.

Wirkstoffgehalt: 98,04%; Säuregehalt: 1,2%; Gehalt an freiem MBT: 0,8%; Schmelzpunkt: 168,4–172,6°C.

**Beispiel 7**

In einem 3 l-Planschliffgefäss mit Überlauf, Rührer, Gaseinleitungsfritte, Tropfrichter und Rückflusskühler wird eine dem stündlichen Durchsatz entsprechende Menge an NaMBT-Lösung vorgelegt und 1 Stunde lang ein Chlor/Luft-Gemisch im Volumenverhältnis 1:20 eingeleitet. Danach wird kontinuierlich NaMBT-Lösung zudosiert, Chlor/-Luft-Gemisch eingeleitet und Reaktionsprodukt über den Überlauf abgenommen. Jede Stunde wird aus dem erhaltenen Reaktionsprodukt das entstandene 2,2'-Dibenzothiazolyldisulfid abgetrennt und aus der Mutterlauge das Rest-Mercaptobenzothiazol mit Salzsäure ausgefällt.

Die nachfolgende Tabelle zeigt die Einzelheiten und Ergebnisse.

Tabelle 1

|   | A | B | C | D | E | F | G | H | I |
|---|---|---|---|---|---|---|---|---|---|
| a | 12 | 14,1 | 0 | 97,4 | 110,4 | 15 | 98,7 | 0,7 | 0,8 |
| b | 11 | 8,8 | 0 | 94,6 | 120 | 50 | 96,5 | 1,8 | 0,9 |
| c | 12 | 8,8 | 3,0 | 98,5 | 115,3 | 36 | 98,9 | 0,6 | 0,3 |
| d | 12 | 8,8 | 5,5 | 99 | 118 | 42 | 99,2 | 0,1 | 0,5 |
| e | 11 | 14,1 | 4,0 | 99 | 103 | 4 | 99,7 | 0 | 0,2 |
| f | 12 | 35,8 | 6,5 | 98 | 105 | 14 | 98,7 | 0 | 1,2 |

## Patentansprüche

1. Verfahren zur Herstellung von 2,2'-Dibenzothiazolyldisulfid durch Oxidation von wässrigen, 8 bis 50 gew.%igen Alkalimetallsalzlösungen des 2-Mercaptobenzothiazols mit Chlor oder Chlor/Luft-Gemischen bei Temperaturen von 15 bis 60°C und pH-Werten von 9 bis 13, dadurch gekennzeichnet, dass die Konzentration des Alkalimetallsalzes des 2-Mercaptobenzothiazols zu keinem Zeitpunkt der Reaktion weniger als 2,5 Gew.% beträgt.

2. Verfahren nach Anspruch 1, dadurch gekennzeichnet, dass die Reaktion kontinuierlich mit einer stationären Konzentration des Alkalimetallsalzes des 2-Mercaptobenzothiazols von 5 bis 25 Gew.% durchgeführt wird.

3. Verfahren nach Anspruch 1, dadurch gekenenzeichnet, dass die Reaktion ansatzweise durchgeführt wird und die Konzentration des Alkalimetallsalzes des 2-Mercaptobenzothiazols am Ende der Reaktion 5 bis 9 Gew.% beträgt.

4. Verfahren nach Anspruch 1, dadurch gekennzeichnet, dass der pH-Wert der Reaktionsmischung 10 bis 12,5 beträgt.

## Claims

1. Process for the preparation of 2,2'-dibenzothiazolyldisulphide by the oxidation of aqueous, 8 to 50% by weight alkali metal salt solutions of 2-mercaptobenzothiazole with chlorine or chlorine/air mixtures at temperatures of from 15 to 60°C and pH values of from 9 to 13, characterized in that the concentration of the alkali metal salt of 2-mercaptobenzothiazole is not less than 2.5% by weight at any time during the reaction.

2. Process according to Claim 1, characterized in that the reaction is carried out continuously with a constant concentration of the alkali metal salt of 2-mercaptobenzothiazole of from 5 to 25% by weight.

3. Process according to Claim 1, characterized in that the reaction is carried out batchwise and the concentration of the alkali metal salt of 2-mercaptobenzothiazole is from 5 to 9% by weight at the end of the reaction.

4. Process according to Claim 1, characterized in that the pH of the reaction mixture is from 10 to 12.5.

## Revendications

1. Procédé de production de disulfure de 2,2'-dibenzothiazolyle par oxidation de solutions aqueuses à 8–50% en poids de sels de métaux alcalins du 2-mercaptobenzothiazole avec du chlore ou des mélanges de chlore et d'air à des températures de 15 à 60°C et à des valeurs de pH de 9 à 13, caractérisé en ce que la concentration du sel de métal alcalin du 2-mercaptobenzothiazole n'a à aucun moment de la réaction une valeur inférieure à 2,5% en poids.

2. Procédé suivant la revendication 1, caractérisé en ce qu'on conduit la réaction en continu avec une concentration stationnaire du sel de métal alcalin du 2-mercaptobenzothiazole de 5 à 25% en poids.

3. Procédé suivant la revendication 1, caractérisé en ce qu'on conduit la réaction en discontinu et la concentration du sel de métal alcalin du 2-mercaptobenzothiazole est de 5 à 9% en poids à la fin de la réaction.

4. Procédé suivant la revendication 1, caractérisé en ce que la valeur du pH du mélange réactionnel est de 10 à 12,5